# EUROPÄISCHE PATENTSCHRIFT

(11) **EP 3 119 444 B1**
(45) Veröffentlichungstag und Bekanntmachung des Hinweises auf die Patenterteilung: **07.08.2019**
(21) Anmeldenummer: 15712526.1
(22) Anmeldetag: 16.03.2015
(51) Int. Cl.: A61L 15/34, A61L 15/58, A61K 9/70

(54) **ÜBERPFLASTER MIT VERBESSERTER VERTRÄGLICHKEIT UND EINER LANGEN HAFTUNGSDAUER UND VERFAHREN ZU SEINER HERSTELLUNG**
FINISHING PLASTER HAVING IMPROVED TOLERABILITY AND A LONG ADHESIVE PERIOD AND METHOD FOR PRODUCING THE SAME
PANSEMENT DE RECOUVREMENT À TOLÉRANCE AMÉLIORÉE ET LONGUE DURÉE D'ADHÉRENCE ET SON PROCÉDÉ DE FABRICATION

(30) Priorität: 19.03.2014 EP 14160657
(43) Veröffentlichungstag der Anmeldung: 25.01.2017
(73) Patentinhaber: LTS Lohmann Therapie-Systeme AG, 56626 Andernach (DE)
(72) Erfinder: RITZDORF, Gerhard, 56598 Hammerstein (DE); HILLE, Thomas, 56567 Neuwied (DE); BOTZEM, Petra, 56626 Andernach (DE); WAUER, Gabriel, 53474 Ahrweiler (DE); FUHRMANN, Marlene, 56829 Kail (DE)
(74) Vertreter: Schweitzer, Klaus
(86) Internationale Anmeldenummer: PCT/EP2015/000579
(87) Internationale Veröffentlichungsnummer: WO 2015/139830

(56) Entgegenhaltungen:
- EP-A1- 0 247 571
- EP-A1- 1 992 363
- WO-A2-02/38136
- WO-A2-2006/036899
- DE-A1-102004 038 285
- DE-A1-102006 054 731

## Beschreibung

Die vorliegende Erfindung betrifft medizinische Produkte, die auf der Haut getragen werden und durch ein Überpflaster fixiert werden. Dies können transdermale therapeutische Systeme (TTS) sein, die neben dem wirkstoffhaltigen Teil zusätzlich ein wirkstofffreies Überpflaster, welches einen speziellen Aufbau aufweist, aber auch Diagnostika oder Kanülen, die durch eine Pflasterbinde fixiert werden. Bekanntlich sind TTS pflasterförmige Arzneimittel, die auf die Haut aufgeklebt werden und zumindest einen Wirkstoff über die gesamte Applikationsdauer abgeben müssen. Es versteht sich von selbst, dass diese medizinischen Produkte gut kleben müssen, da ansonsten keine Wirkstoffabgabe an die Haut erfolgen kann, das Diagnostikum nicht verlässlich arbeitet oder die Kanüle verrutscht. Gleichzeitig müssen Hautirritationen vermieden werden, das heißt, die Eigenklebrigkeit der TTS, Diagnostikums oder Kanülenfixierpflasters darf nur so hoch wie unbedingt nötig sein.

Bekanntlich versteht man unter Kleben das Verbinden zweier Oberflächen durch einen Klebstoff, der die Oberflächen infolge Adhäsion und Kohäsion miteinander verbindet. Der Klebstoff muss dabei die jeweiligen Oberflächen benetzen. Daraus folgt, dass für gute Klebrigkeit nicht nur der Klebstoff, sondern auch die Beschaffenheit der Oberflächen von Bedeutung sind. Nun haben verschiedene Menschen, besonders mit fortschreitendem Alter Häute mit verschiedenen Oberflächen, wobei die einzelnen Hauttypen mehr oder weniger empfindlich auf Hautreizungen reagieren. Obwohl dieser Bedarf der Fachwelt bekannt ist, gibt es noch keine medizinischen Produkte, die dieser Fragestellung gerecht werden.

Pflaster aus Polyacrylaten über eine Tragedauer bis zu mindestens 7 Tagen genutzt werden können, sind z.B. aus der US 2009/0130190 A1 bekannt. Solche Pflaster führen in der Regel insbesondere bei langer Tragedauer beim Wiederablösen zu merklichen Schmerzen für die betroffene Haut und erhöhten sichtbaren Rückständen an der Haut. Hinzu kommt die gängige Nutzung von Flächengewichten von über 80 g/m² mit dem Nachteil von höheren Restmonomergehalten im gesamten Pflaster. Dies rührt daher, dass bei Überpflastern, die für mindestens 7 Tage ausgelegt sind, oft saure Polyacrylatkleber (Monomere weisen freie Säurefunktionen auf) ohne Zusätze wie Neutralöle genutzt werden, und daher eine sehr starke Bindung zur Haut eingehen.

WO 2009/009649 beschreibt ein transdermales therapeutisches System bestehend aus einem zentralen Kompartiment und einem wirkstofffreien Überpflaster, das das zentrale Kompartiment fixiert und allseitig überragt. Das Überpflaster besteht aus einer wasserdampfdurchlässigen Rückschicht und einer haftklebenden Polymerschicht. Die haftklebende Polymerschicht umfasst beispielsweise Polyisobutylen, Styren-Isopren-Styren-Block-Coolymere oder Styren-Butadien-Styren-Block-Copolymere.

Als Hautkontaktschichten werden bei wirkstoffhaltigen Silikonklebern oft amin-resistente Silikonkleber genutzt. Diese weisen oft den Nachteil auf, dass diese aufgrund ihrer Amin-Resistenz geringere Bindung zur Haut aufweisen, da alle endständigen Silanolgruppen mit Methylgruppen "gecapped" sind. Daher ist es mit den gängigen Silikonklebern oft sehr schwer eine Tragedauer von mindestens 7 Tagen oder darüber hinaus zu erreichen. Zusätzlich weisen amin-resistente Polysiloxankleber geringere Wasserdampfdurchlässigkeitswerte auf, als dies bei nicht amin-resistenten Versionen der Polysiloxankleber der Fall ist, so dass der Okklusionseffekt bei diesen geringer ausfällt. Bei Nutzung eines Überpflasters, welches mindestens 7 Tage an der Haut mit guter Verträglichkeit haftet, kann dieser Nachteil ausgeglichen werden. Als weiterer Aspekt wird oft als Backing Layer bei wirkstoffhaltigen Silikonpflastern eine einseitig silikonisierte Folie genutzt (z.B. 19 oder 23 µm PET Folie einseitig silikonisiert) Hierbei tritt der nachteilige Effekt auf, dass diese auch auf der nicht-silikonisierten Seite Silikonreste aufweist, da die Rollen in sich gewickelt werden, so dass die nicht silikonisierte Seite in Kontakt zur silikonisierten Seite tritt. Daher ist die Haftung gegenüber der wirkstoffhaltigen Silikonkleberschicht gut, aber gegenüber eines auf Acrylatklebern basierenden Überpflasters geringer. Es kann daher zu verfrühten Ablösungen (bzw. schlechter Haftung) zwischen Überpflaster und wirkstoffhaltiger Matrix mit einer Trennschicht aus einseitig silikonisierter Folie (bzw. Backing Layer) kommen. Mit einem auf nicht amin-resistenten Silikonklebern basierenden wirkstofffreien Überpflasters kann dieser Nachteil ausgeglichen werden.

Aufgabe der vorliegenden Erfindung war daher die Bereitstellung eines wirkstofffreien Überpflasters, welches die Nachteile des Standes der Technik nicht oder nur in weit geringerem Ausmaß aufweist.

Diese Aufgabe wird gelöst durch medizinische Produkte mit einem zentralen Kompartiment und einem wirkstofffreien, das zentrale Kompartiment allseitig überragendem Überpflaster, denn durch diese Konstruktion wird den Anforderungen an standardisierte Produktionsverfahren Rechnung getragen, denn der wesentliche Teil kann in großen Chargen gefertigt werden, während die Varianten, die durch die verschiedenen Hauttypen erforderlich sind, durch unterschiedliche Überpflaster möglich werden. Für den klebenden Teil im direkten Hautkontakt werden Silikonkleber mit erhöhter Wasserdampfdurchlässigkeit und damit verbundenem geringeren Okklusionseffekt benutzt. Der Silikonkleber kann zudem Silikonöle als Tackifier enthalten.

Als Rückschicht 1 kann ein elastisches, Wasserdampf durchlässiges Gewebe genutzt werden, um dem Pflaster eine gewisse Flexibilität zu geben. Sie ist bevorzugt aus Polyestergewebe und kann zwecks besserer Unterscheidung der Überpflastertypen entsprechend verschieden gefärbt vorliegen. Vorzugsweise wird ein in Kett- und Schussrichtung dehnbares bidirektional elastisches Polyestergewebe, insbesondere ein Polyethylenterephthalat-Gewebe verwendet. Die Dehnbarkeit dieses Gewebes beträgt ≥ 35 %, vorzugsweise ≥ 45 %, insbesondere ca. 50 % in Längsrichtung und ≥ 25 %, vorzugsweise ≥ 35 % in Querrichtung, gemessen nach DIN 61632. Das Flächengewicht beträgt ≥ 100 g/m² , vorzugsweise ≥ 120 g/m², insbesondere ca. 130 g/m². Die Karl Otto Braun GmbH & Co. KG (KOB) ist ein Hersteller solcher Gewebe.

Ein weiterer Bestandteil der Erfindung ist die Nutzung von möglichst geringen Beschichtungsgewichten.

Die Silikonklebervariante zeigt den Vorteil, dass hier überhaupt keine Restmonomere vorhanden sind.

Dieses Überpflaster dient vorzugsweise zur Fixierung von wirkstoffhaltigen Matrixpflastern bestehend aus einer wirkstoffhaltigen Kleberschicht, in der als Kleber amin-resistente Polysiloxankleber, Polyacrylatkleber oder Polyisobutylenkleber Anwendung finden. Diese Kleberschicht ist im direkten Hautkontakt. Als Trennschicht zum wirkstofffreien Überpflaster findet eine Sperrschicht aus PET Folie Anwendung, die bei Silikonklebersystemen meist einseitig silikonisiert ist. Das Überpflaster wird mit überlappenden Rand aufgebracht, so dass der überlappende wirkstofffreie Rand des Überpflasters eine Fixierung über eine Tragedauer von mindestens 7 Tagen gewährleistet.

Die vorliegende Erfindung betrifft daher ein medizinisches Produkt gemäß Anspruch 1.

Die Zusammensetzung der Schicht 2 ist abhängig von den Bedürfnissen des speziellen Hauttyps des Patienten so gewählt, dass das medizinische Produkt für eine Fixierdauer von mindesten 7 Tagen genutzt werden kann (Tragedauer ohne vollständige Ablösung des Pflasters von mindestens 7 Tagen auf menschlicher intakter Haut) und dabei den Anforderungen an eine gute Hautverträglichkeit gerecht wird.

Vorzugsweise handelt es sich um ein transdermales, therapeutisches System (TTS), worin das Kompartiment 3 aus
c) mindestens einer haftklebenden wirkstoffhaltigen Polymerschicht (5), die nach Abziehen der wiederablösbaren Schutzschicht (6) direkten Hautkontakt hat, und
d) einer die Schicht 5 bedeckenden Trennschicht (4), vorzugsweise aus Polyester, besteht.

Bei dem erfindungsgemäßen medizinischen Produkt kann es sich auch um ein Diagnostikum handeln, bei dem die Diagnosevorrichtung im Kompartiment 3 lokalisiert ist; oder es kann sich um ein Kanülenfixierpflaster handeln, bei dem die Kanüle im Kompartiment 3 lokalisiert ist. Aus der US 2011/144470 A1 sind Diagnosevorrichtungen bekannt, die auf die Haut geklebt werden. Fixiersysteme zur Befestigung von Kanülen auf der Haut sind beispielsweise in der WO 87/07164 A1 beschrieben.

Die bevorzugten TTS sind auf die Haut aufzubringende Arzneiformen mit dem Aussehen traditioneller Pflaster, die über die Haut abzugebende Arzneistoffe enthalten. Ein TTS kann einen oder mehrere Arzneistoffe enthalten, die in vorausbestimmter Rate kontinuierlich über einen festgesetzten Zeitraum an einen festgelegten Anwendungsort abgegeben werden ("Heilmann, Klaus: Therapeutische Systeme Konzept und Realisation programmierter Arzneiverabreichung", 4. Auflage, Ferdinand Enke Verlag Stuttgart, 1984, S. 26).

Die wirkstoffhaltige Haftkleberschicht der TTS besteht aus einer Polymermatrix mit einem Grundpolymer und gegebenenfalls den üblichen Zusätzen. Geeignete Polymere sind Silikone, Kautschuk, kautschukähnliche synthetische Homo-, Co- oder Blockpolymere, Polyacrylsäureester und deren Copolymere und Ester von hydriertem Kolophonium. Grundsätzlich kommen alle Polymere in Frage, die bei der Herstellung von Haftklebern eingesetzt werden und physiologisch unbedenklich sind. Geeignet sind z.B. sind solche, die als Blockcopolymere auf Basis von Styrol und 1,3-Dienen, Polyisobutylenen oder Polymeren und Copolymeren aus Acrylat und/oder Methacrylat bestehen. Von den Blockcopolymeren auf Basis von Styrol und 1,3-Dienen finden z.B. lineare Styrol-Isopren-Styrol Blockcopolymere Verwendung.

Als Polymere auf Acrylatbasis sind beispielsweise Acrylatcopolymere aus 2-Ethylhexylacrylat, Vinylacetat und Acrylsäure mit bzw. ohne Titanchelatester bevorzugt. Als Ester von hydriertem Kolophonium werden vorzugsweise dessen Methyl- und Glycerinester verwendet.

Die Art der als Zusätze möglichen Weichmacher und Klebrigmacher hängt vom verwendeten Polymer ab. Die in Frage kommenden physiologisch unbedenklichen Substanzen sind bekannt. Die Eigenklebrigkeit der Haftkleberschicht muss den dauernden Kontakt zur Haut sicherstellen. Sie kann im Hotmelt-Verfahren, als Lösung oder als Dispersionshaftkleber auf die Rückschicht aufgebracht werden.

Als Wirkstoffe werden Substanzen verwendet, die ohne oder mit Resorptionsvermittler auf der Haut appliziert eine lokale oder systemische Wirkung hervorrufen.

Stoffe mit lokaler Wirkung sind zum Beispiel Antitranspirantia, Fungizide, Bactericide und Bacteriostatica.

Stoffe mit systemischer Wirkung sind beispielsweise Antibiotika, Hormone, Antipyretica, Antidiabetica, Koronardilatatoren, herzwirksame Glycoside, Spasmolytica, Antihypertonica, Psychopharmaka, Migränemittel, Corticoide, Analgetica, Antikontrazeptiva, Antirheumatica, Anticholinergica, Sympatolytica, Sympatomimetica, Vasodilatatoren, Anticoagulantien und Antiarrhythmika.

Von dem verwandten Polymer und dem Wirkstoff abhängige mögliche Zusätze sind Weichmacher, Klebrigmacher, Stabilisatoren, Trägerstoffe, diffusions- und penetrationsregulierende Zusätze oder Füllstoffe. Die in Frage kommenden physiologisch unbedenklichen Substanzen sind bekannt.

Die Schicht 2 kann 0 bis 5 % (g/g) eines Neutralöls, bezogen auf das trockene Gewicht dieser Schicht, als Verträglichmacher enthalten. Schicht 2 kann auch 0,5 bis 5 % (g/g) Dexpanthenol, bezogen auf das trockene Gewicht dieser Schicht, enthalten.

Schicht 2 umfasst ein nicht amin-resistente Polysiloxanpolymere, bei welchen nach der Polykondensation des Harz-Anteils und der Polydimethylsiloxanolgruppen die noch freien restlichen Silanolgruppen nicht mit Methylgruppen "gecapped" wurden, dem 0 bis 4 % (g/g) Silikonöl, bezogen auf das trockene Gewicht dieser Schicht, zugesetzt werden.

Insbesondere weist das bevorzugte TTS die genannten Schichten jeweils einmal auf, und es besteht bevorzugt aus den genannten Schichten. Die beiden genannten haftklebenden Schichten können bezüglich ihrer Polymerzusammensetzung und/oder Dicke gleich oder verschieden sein. Die auf der Haut aufzubringende haftklebende Polymerschicht ist bevorzugt zusätzlich mit einer Schutzschicht bei Acrylat und Polyisobutylenklebern aus z.B. silikonisiertem Papier oder silikonisierter Folie (z.B. PET-Folie) versehen. Bei Silikonklebern findet eine mit Fluorpolymer beschichtete PET Schutzschicht ihre Anwendung. Die einzelnen Pflaster sind in üblicher Weise verpackt, z.B. in einem Vierrandsiegelbeutel aus Verbundpackstoff aus Polyacrylnitril, Aluminiumfolie und Papier.

Die im Hautkontakt stehende haftklebende und hautfreundliche Polymerschicht 2 enthält nicht amin-resistente Polysiloxane. Besonders bevorzugt besteht diese Schicht aus einem nicht amin-resistenten Polysiloxan, dass zur Erhöhung des Tacks kleine Mengen an Silikonöl enthalten kann.

Es ist ein besonderes Merkmal des erfindungsgemäßen Überpflasters, dass die im Hautkontakt stehende, haftklebende und hautfreundliche Polymerschicht 2 nicht amin-resistente Polysiloxane mit oder ohne Silikonöl zur Erhöhung des Tacks in sehr kleinen Mengen enthalten kann und in einer bevorzugten Ausführungsform auch enthält.

Die undurchlässige Trennschicht (4) zwischen wirkstoffhaltiger Schicht und Überpflaster kann aus verschiedenen Polymeren bestehen. Bevorzugt sind hier Polyester und von diesen insbesondere Polyethylenterephthalat. Die Schicht 4 findet Anwendung, sobald eine wirkstoffabgebende Einheit von der wirkstofffreien Überpflastereinheit getrennt werden soll.

Gut haftende Polymere sind beispielsweise Polyacrylate. Besonders gut kleben auf der Haut solche Polyacrylate, die durch Verwendung von freier Acrylsäure hergestellt wurden. Jedoch führen diese zu Hautreizungen und merklichen Schmerzen beim Wiederablösen. In Trageversuchen wurden Polyacrylate genutzt ohne freie Acrylsäure, um eine Tragedauer bei gleichzeitig guter Hautverträglichkeit nachzuweisen. Diese Versuche im Rahmen von 2²-faktor Versuchsplänen waren erfolgreich. Daher können bevorzugt Polymere mit Säurezahlen von < 1 genutzt werden.

Die gleichen Ausführungen gelten grundsätzlich auch für Polysiloxane, wobei bei diesen Polymeren saure Monomere aufgrund ihres anderen chemischen Aufbaus nicht vorkommen. Sie haften daher nur dann zuverlässig über eine Dauer von mindestens 7 Tagen, wenn sie mit nicht amin-resistenten Polysiloxanklebern "Standard PSA" hergestellt werden, um genügend Hydrophile Strukturen mit erhöhter Bindungsaffinität zur Haut aufzuweisen.

Es hat sich gezeigt, dass ein TTS mit einem erfindungsgemäßen wirkstofffreien Überpflaster eine bedeutend geringere Neigung zur Kristallisation des Wirkstoffs / der Wirkstoffe in der haftklebenden wirkstoffhaltigen Polymerschicht 5 aufweist. Es neigt außerdem in weit geringerem Maße zu kaltem Fluss. Dies führt dazu, dass das TTS seltener an der Verpackungsoberfläche kleben bleibt und sich daher leichter aus dem Siegelrandbeutel entnehmen lässt.

Die Erfindung betrifft daher auch die Verwendung eines wirkstofffreien Überpflasters zur Verhinderung der Kristallisation des Wirkstoffs / der Wirkstoffe in der wirkstoffhaltigen Polymerschicht 5 eines TTS und die Verwendung eines wirkstofffreien Überpflasters zu Verminderung des kalten Flusses in einem TTS.

Die Erfindung betrifft auch Verfahren zur Herstellung des erfindungsgemäßen medizinisches Produkts gemäß Anspruch 7.

Das W.E. Robert Skin Type Classification System wurde in jüngerer Zeit eingeführt, um eine Einteilung des Hauttyps z.B. bei kosmetischen Behandlungen zu ermöglichen (siehe z.B. Roberts, J. Drugs Dermatol. 2008 May; 7(5): 452 - 456 und Robert, Dermatol. Clin. 2009 Oct; 27(4): 529 - 533). Es handelt sich dabei um ein vierteiliges System, das nicht nur die Eigenschaften des Hauttyps bestimmt, sondern auch Informationen zur Prognose der Hautreaktionen liefert.

Die Erfindung betrifft auch ein Kit-of-parts gemäß Anspruch 8.

Mit dieser bevorzugten Ausführungsform erhält der Anwender einen "Baukasten", der ihm erlaubt das in einem standardisierten Verfahren gefertigte und strenge pharmazeutische Auflagen erfüllende Kompartiment 3 mit einem geeigneten Überpflaster zu kombinieren, damit das medizinische Produkt den Anforderungen an eine Fixierdauer von mindesten 7 Tagen genutzt werden kann und dabei den Anforderungen an eine gute Hautverträglichkeit gerecht wird.

Die Erfindung betrifft weiterhin ein Kit-of-parts gemäß Anspruch 10.

Auch mit diesem Kit erhält der Anwender einen "Baukasten", der ihm erlaubt das in einem standardisierten Verfahren gefertigte und strenge pharmazeutische Auflagen erfüllende Kompartiment 3 mit einem geeigneten Überpflaster zu kombinieren, damit das medizinische Produkt den Anforderungen an eine Fixierdauer von mindestens 7 Tagen genutzt werden kann und dabei den Anforderungen an eine gute Hautverträglichkeit gerecht wird.

Die Erfindung betrifft schließlich die Verwendung eines vorstehend beschriebenen Kit-of-parts gemäß einem der Ansprüche 8 bis 11.

Das Kompartiment 3 ist haftklebend und hat nach Abziehen der wiederablösbaren Schutzschicht (6) direkten Hautkontakt, bei welchem ein auf einen Zwischenträger auflaminiertes wirkstofffreies Überpflaster nach Abziehen des Zwischenträgers auf ein Kompartiment 3 aufgeklebt wird.

Die nachfolgenden besonders bevorzugten Ausführungsformen der vorliegenden Erfindung, die Zeichnungen und die Ausführungsbeispiele dienen zur näheren Erläuterung der Erfindung, ohne diese darauf beschränkt wäre. Vielmehr ist jede für den Fachmann sinnvolle Kombination von (einzelnen) Merkmalen, unabhängig davon, ob sie in der Beschreibung, den Beispielen oder Ansprüchen genannt sind, von der vorliegenden Erfindung umfasst.

In der Zeichnung zeigt:
Fig. 1 einen Schnitt durch das erfindungsgemäße medizinische Produkt.
Fig. 2 einen Schnitt durch das erfindungsgemäße bevorzugte TTS.

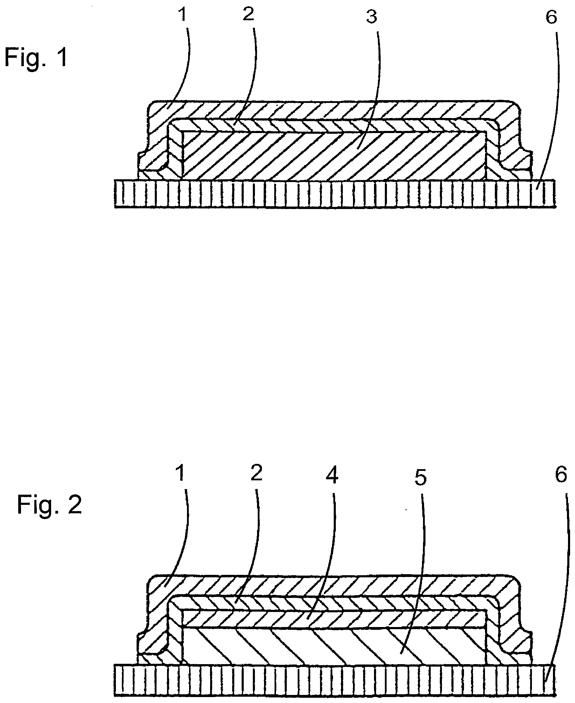

In Fig. 1 und 2 sind die Schichtdicken der Deutlichkeit wegen übertrieben dargestellt. In Fig. 1 ist das haftklebende zentrale Kompartiment (3) schematisch mit einem rechteckigen Querschnitt dargestellt. Der tatsächliche Querschnitt kann davon abweichen.

Wie in Fig. 1 zu sehen ist, ruht Kompartiment 3 auf der wiederablösbaren Schutzschicht (6) und wird von dem aus der wasserdampfdurchlässigen Rückschicht (1) und der haftklebenden wirkstofffreien Polymerschicht (2) gebildeten Überpflaster bedeckt. Es ist zu erkennen, dass Kompartiment 3 von der Rückschicht 1 und der Polymerschicht 2 allseitig überragt wird.

Wie in Fig. 2 zu sehen ist, umfasst das TTS eine haftklebende wirkstoffhaltige Polymerschicht (5), die auf der wiederablösbaren Schutzschicht (6) ruht und von dem aus der wasserdampfdurchlässigen Rückschicht (1) und der haftklebenden wirkstofffreien Polymerschicht (2) gebildeten Überpflaster bedeckt wird. Die Trennschicht (4) trennt dabei die wirkstoffhaltige Schicht 5 von der haftklebenden wirkstofffreien Polymerschicht (2). Es ist zu erkennen, dass aus den Schichten 4 und 5 gebildetes Laminat von der Rückschicht 1 und der Polymerschicht 2 allseitig überragt wird.

In bevorzugten Ausführungsformen umfasst die gut hautverträgliche wirkstofffreie Polymerschicht (2) (hier und im Folgenden angegebene prozentuale Gehaltsgrößen sind Massenanteile [w/w]):
- einem nicht amin-resistenten Polysiloxankleber "Standard PSA" mit einem optionalen Silikonölanteil (z.B. Silikonöl 12500 cST) zur Erhöhung des Tacks von < 4 % und einem Flächengewicht von < 80g/m².

In besonders bevorzugten Ausführungsformen umfasst die gut hautverträgliche wirkstofffreie Polymerschicht (2):
- einen nicht amin-resistenten Polysiloxankleber "Standard PSA" mit einem optionalen Silikonölanteil (z.B. Silikonöl 12500 cST) zur Erhöhung des Tacks von ≤ 1 % und einem Flächengewicht von ≤ 70g/m².

Die Trennschicht (4) zwischen Wirkstoff abgebender Einheit 5 und dem aus den Schichten 1 und 2 gebildeten Überpflaster besteht bevorzugt aus Polyethylenterephthalat (PET) mit einer Dicke von 19 - 23 µm. Sie kann bei Nutzung von Polysiloxanklebern in den Schichten 2 oder 5 silikonisiert sein.

### Herstellungsbeispiele:

### Beispiele 1 - 5a (DoE1) mit Polyacrylatkleber + Neutralöl (nicht erfindungsgemäß):

Herstellung der Masse für das wirkstofffreie Überpflaster mit Polyacrylatkleber + Neutralöl:
Eine Vinylacetat-Acrylat-copolymerisat Lösung in Etylacetat/Ethanol/Heptan mit einem Feststoffanteil von 40 - 43 % [w/w] wird mit einer Lösung von Neutralöl (Miglyol) versetzt, und der Gesamtfeststoffanteil der Lösung mit Ethanol auf 40 % eingestellt.

Dann wird gerührt, bis eine homogene Lösung entsteht.

Diese Kleberlösung (Masse) wird für die Herstellung der wirkstofffreien, der Haut zugewandten Seite (Schicht 2), des Überpflasters genutzt.

Beschichtung und Trocknung der Masse auf Zwischenträger:
Die Masse wird so auf ein silikonisiertes PE-Papier beschichtet, dass nach Abdampfen der Lösemittel im Trockenschrank oder Trockenkanal bei maximal 100 °C eine Kleberschicht mit einem Flächengewicht von 50 - 110 g/m² entsteht.

Die eingesetzten Zusammensetzungen der Kleberlösungen für das Überpflaster je Formulierung betrugen bei den DoE1 (Design of Experiment) Versuchen:

**Tabelle 1:**

| Beispiel | Vinylacetat-Acrylat-copolymerisat Lösung 42,1 % (z.B. ®DURO-TAK 387-2515)[g] | Neutralöl (®Miglyol 812)[g] | Ethanol[g] | Besch ichtungs-gewicht nach dem Trocknen bei ca. 10 - 12 Min bei 60°C [g/m²] |
|---|---|---|---|---|
| Beispiel 1 | 181,6 | 1,6 | 11,9 | 81,1 |
| Beispiel 2 | 368,5 | 0,82 | 30,6 | 52,5 |
| Beispiel 3 | 368,5 | 0,82 | 30,6 | 114,4 |
| Beispiel 4 | 352,2 | 7,8 | 30,2 | 49 |
| Beispiel 5 | 352,2 | 7,8 | 30,2 | 114,1 |
| Beispiel 5a | 181,6 | 0,8 | 11,9 | 75 |

### Beispiele 6 - 10 (DoE2) mit Polyacrylatkleber + Dexpanthenol (nicht erfindungsgemäß):

Herstellung der Masse für das wirkstofffreie Überpflaster mit Polyacrylatkleber + Dexpanthenol:
Eine Vinylacetat-Acrylat-copolymerisat Lösung in Ethylacetat/Ethanol/Heptan mit einem Feststoffanteil von 40 - 43 % [w/w] wird mit Dexpanthenol versetzt, und der Gesamtfeststoffanteil der Lösung mit Ethanol auf 40 % eingestellt.

Dann wird gerührt, bis eine homogene Lösung entsteht.

Diese Kleberlösung (Masse) wird für die Herstellung der wirkstofffreien, der Haut zugewandten Seite (Schicht 2), des Überpflasters genutzt.

Beschichtung und Trocknung der Masse auf Zwischenträger:
Die Masse wird so auf ein silikonisiertes PE-Papier beschichtet, dass nach Abdampfen der Lösemittel im Trockenschrank oder Trockenkanal bei maximal 100 °C eine Kleberschicht mit einem Flächengewicht von 50 - 110 g/m² entsteht.

Die eingesetzten Zusammensetzungen der Kleberlösungen für das Überpflaster je Formulierung betrugen bei den DoE2 (Design of Experiment) Versuchen (Beispiele 6 - 10):

**Tabelle 2:**

| Beispiel | Vinylacetat-Acrylat-copolymerisat Lösung 42,1 % (z.B. ®DURO-TAK 387-2515)[g] | Dexpanthenol [g] | Ethanol [g] | Beschichtungsgewicht nach dem Trocknen bei ca. 10 - 12 Min bei 60°C [g/m²] |
|---|---|---|---|---|
| Beispiel 6 | 180,4 | 2,1 | 12,5 | 80,7 |
| Beispiel 7 | 368,8 | 0,8 | 20,5 | 47,8 |
| Beispiel 8 | 368,8 | 0,8 | 20,5 | 119,2 |
| Beispiel 9 | 352,9 | 7,9 | 30,2 | 50,6 |
| Beispiel 10 | 352,9 | 7,9 | 30,2 | 113,5 |

### Beispiele 11 - 15 (DoE3) und 16 - 20 (DoE4) mit nicht amin-resistentem Polysiloxankleber + Silikonöl:

Herstellung der Masse für das wirkstofffreie Überpflaster mit nicht amin-resistentem Polysiloxankleber + Silikonöl:
Eine nicht amin-resistente Polysiloxankleber Lösung in Heptan (alternativ Ethylacetat) mit einem Feststoffanteil von 50 - 70 % [w/w] wird mit Silikonöl versetzt, gegebenenfalls mit n-Heptan (bzw. bei in Ethylacetat gelösten nicht amin-resistenten Polysiloxanklebern mit Ethylacetat) auf 60 - 70 % verdünnt und dann gerührt, bis eine homogene Lösung entsteht.

Diese Kleberlösung (Masse) wird für die Herstellung der wirkstofffreien, der Haut zugewandten Seite (Schicht 2), des Überpflasters genutzt.

Beschichtung und Trocknung der Masse auf Zwischenträger:
Die Masse wird so auf eine mit Fuorpolymer beschichtete PET Folie (Schutzschicht bzw. Zwischenträger), dass nach Abdampfen der Lösemittel im Trockenschrank oder Trockenkanal bei maximal 100 °C eine Kleberschicht mit einem Flächengewicht von 50 - 80 g/m² entsteht.

Die eingesetzten Zusammensetzungen der Kleberlösungen für das Überpflaster je Formulierung betrugen bei den DoE3 (Design of Experiment) Versuchen:

**Tabelle 3:**

| Beispiel | Nicht amin-resistente Polysiloxan Kleber Lösung 60 % (z.B. ®BIO-PSA 7-4501) [g] | Silikonöl [g] | n-Heptan [g] | Beschichtungsgewicht nach dem Trocknen bei ca. 10Min bei 50°C [g/m²] |
|---|---|---|---|---|
| Beispiel 11 | 323,1 | 1,0 | 0,6 | 54,4 |
| Beispiel 12 | 323,1 | 1,0 | 0,6 | 85,9 |
| Beispiel 13 | 310,9 | 7,9 | 5,2 | 51,9 |
| Beispiel 14 | 310,9 | 7,9 | 5,2 | 81,6 |
| Beispiel 15 | 160,5 | 1,0 | 0,7 | 65,9 |

Die eingesetzten Zusammensetzungen der Kleberlösungen für das Überpflaster je Formulierung betrugen bei den DoE4 (Design of Experiment) Versuchen:

**Tabelle 4:**

| Beispiel | Nicht amin-resistente PolysiloxanKleber-Lösung 61 % (z.B. ®BIO-PSA 7-4601) [g] (evt. Abweichender Feststoffgehalt) | Silikonöl [g] | n-Heptan [g] | Beschichtungsgewicht nach dem Trocknen bei ca. 10 Min bei 50°C [g/m²] (Viskosität der Beschichtungsmasse in dPas) |
|---|---|---|---|---|
| Beispiel 16 | 311,5 | 1,0 | 3,0 | 59,5 |
| Beispiel 17 | 311,5 | 1,0 | 3,0 | 85,6 |
| Beispiel 18 | 306,4 | 7,9 | 10,2 | 59,2 |
| Beispiel 19 | 306,4 | 7,9 | 10,2 | 84,2 |
| Beispiel 20 | 167,4 | 1,0 | 3,4 | 72,6 |
| Beispiel 21 | 160,5 (60 %) | 0,7 | 0,7 | 69 (5 dPas) |
| Beispiel 22 | 160,5 (65 %) | 0,7 | 0,7 | 70 (8 dPas) |
| Beispiel 23 | 160,5 (67 %) | 0,7 | 0,7 | 70 (9,5 dPas) |
| Beispiel 24 | 160,5 (69 %) | 0,7 | 0,7 | 70 (15 dPas) |

Die getrocknete Kleberoberfläche wird mit einer wasserdampfdurchlässigen Rückschicht 1, z.B. Polyestergewebe, elastisch abgedeckt. Dies ist beispielsweise ein in Kett- und Schussrichtung dehnbares bidirektional elastisches Polyethylenterephthalat-Gewebe, dessen Dehnbarkeit ca. 50 % in Längsrichtung, ≥ 35 % in Querrichtung (gemessen nach DIN 61632) und dessen Flächengewicht ca. 130 g/m² beträgt (Hersteller: Karl Otto Braun GmbH & Co. KG (KOB)).

Aus den so entstehenden Laminaten bestehend aus Zwischenträger, getrockneter Polymerschicht 2 und Rückschicht 1 werden nun Überpflaster geeigneter Größe ausgestanzt, um diese z.B. bei Anwendung in Kombination mit einer Wirkstoff abgebenden Schicht 5 auf die Trennschicht 4 zu kleben. Die Stanzung des Überpflasterlaminates erfolgt in der Weise, dass alle Schichten außer dem Zwischenträger durchtrennt werden. Die Größe des Überpflasters in Vergleich zur wirkstoffabgebenden Einheit inklusive der Trennschicht bestimmt hierbei maßgeblich die Größe des überlappenden Randbereiches, der die Fixierung in direktem Hautkontakt ermöglicht. Die Breite des überlappenden Randbereiches sollte nicht weniger als 0,4 cm betragen, um eine ausreichende Fixierung über 7 Tage zu gewährleisten.

### Lagertests

Zur Herstellung eines TTS für die Lagertests wurde eine 16 cm² große und 0,1 mm dicke, 3,2 mg Estradiol-Hemihydrat und 11,2 mg Norethisterol enthaltende Vinylacetat-Acrylat-Copolymerisat-Matrix mit einem allseitig überstehenden Überpflaster aus Acylatkleber und bidirektional elastischem PES-Gewebe abgedeckt. Die so hergestellten Pflaster wurden in Surlyn- bzw. PET/AL/PAN (Barex)-Siegelrandbeutel verpackt.

Die verpackten Pflaster wurden dann 1,5, 3, 4,5 oder 6 Monate lang bei 40 °C und normaler Luftfeuchtigkeit bzw. bei 40 °C und erhöhter Luftfeuchtigkeit gelagert. Anschließend wurden sie aus den Siegelrandbeuteln entnommen. Dabei wurde das Verkleben mit dem Beutelmaterial und der kalte Fluss beurteilt. Außerdem wurden die Pflaster mikroskopisch auf Kristalle untersucht.

### Ergebnisse und Diskussion

**Tabelle 5:**

| | 40 °C | 40 °C | 40 °C und erhöhte Feuchtigkeit | 40 °C und erhöhte Feuchtigkeit |
|---|---|---|---|---|
| | Surlyn-Beutel | Barex-Beutel | Surlyn-Beutel | Barex-Beutel |
| 0 Mon. | keine Kristalle sichtbar | keine Kristalle sichtbar | keine Kristalle sichtbar | keine Kristalle sichtbar |
| 1,5 Mon. (n=3) | leicht entnehmbar; keine Kristalle sichtbar | leicht entnehmbar; keine Kristalle sichtbar | leicht entnehmbar; keine Kristalle sichtbar | leicht entnehmbar; keine Kristalle sichtbar |
| 3 Mon. (n=3) | leicht entnehmbar; keine Kristalle sichtbar | leicht entnehmbar; keine Kristalle sichtbar | leicht entnehmbar; keine Kristalle sichtbar | leicht entnehmbar; keine Kristalle sichtbar |
| 4,5 Mon. (n=3) | leicht entnehmbar; keine Kristalle sichtbar | leicht entnehmbar; keine Kristalle sichtbar | leicht entnehmbar; keine Kristalle sichtbar | leicht entnehmbar; keine Kristalle sichtbar |
| 6 Mon. (n=6) | leicht entnehmbar; in einem der 6 Beutel war 1 runder Kristall sichtbar | leicht entnehmbar; keine Kristalle sichtbar | leicht entnehmbar; keine Kristalle sichtbar | leicht entnehmbar; keine Kristalle sichtbar |

### Schlussfolgerung

1. Durch das Überpflaster kann der kalte Fluss erfolgreich gestoppt werden.
2. Die Kristallisation der Wirkstoffe kann durch das Überpflaster weitgehend verhindert werden. Nach 6 Monaten bei 40 °C und normaler Luftfeuchtigkeit wurde nach 6 Monaten nur in einem Fall ein Kristall gefunden. In Pflastern, die bei 40 °C und erhöhter Luftfeuchtigkeit gelagert wurden, konnten überhaupt keine Kristalle nachgewiesen werden.

## Patentansprüche

1. Medizinisches Produkt, bestehend aus einem zentralen Kompartiment (3) und einem wirkstofffreien Überpflaster aus
a) einer wasserdampfdurchlässigen Rückschicht (1) und
b) einer haftklebenden wirkstofffreien Polymerschicht (2),
worin das Kompartiment 3 haftklebend ist und nach Abziehen der wiederablösbaren Schutzschicht (6) direkten Hautkontakt hat,
**dadurch gekennzeichnet, dass** das Überpflaster das zentrale Kompartiment (3) fixiert und allseitig überragt und dass die wirkstofffreie Polymerschicht (2) nicht amin-resistente Polysiloxanpolymere umfasst, bei welchen nach der Polykondensation des Harz-Anteils und der Polydimethylsiloxanolgruppen die noch freien restlichen Silanolgruppen nicht mit Methylgruppen "gecapped" wurden.

2. Medizinisches Produkt gemäß Anspruch 1, **dadurch gekennzeichnet, dass** es sich um ein transdermales, therapeutisches System (TTS) handelt, worin das Kompartiment 3 aus
a) mindestens einer haftklebenden wirkstoffhaltigen Polymerschicht (5), die nach Abziehen der wiederablösbaren Schutzschicht (6) direkten Hautkontakt hat, und
b) einer die Schicht (5) bedeckenden Trennschicht (4), vorzugsweise aus Polyester,
besteht.

3. Medizinisches Produkt gemäß Anspruch 1, **dadurch gekennzeichnet, dass** es sich um ein Diagnostikum handelt und die Diagnosevorrichtung im Kompartiment (3) lokalisiert ist.

4. Medizinisches Produkt gemäß Anspruch 1, **dadurch gekennzeichnet, dass** es sich um ein Kanülenfixierpflaster handelt und die Kanüle im Kompartiment (3) lokalisiert ist.

5. Medizinisches Produkt gemäß einem der Ansprüche 1 bis 4, **dadurch gekennzeichnet, dass** die Schicht (2) 0 bis 4 % (w/w) Silikonöl, bezogen auf das trockene Gewicht dieser Schicht, enthält.

6. Medizinisches Produkt gemäß einem der Ansprüche 1 bis 5, **dadurch gekennzeichnet, dass** die Rückschicht (1) ein elastisches, für Wasserdampf durchlässiges Gewebe, bevorzugt ein Polyestergewebe, umfaßt.

7. Verfahren zur Herstellung eines medizinisches Produkts gemäß einem der Ansprüche 1 bis 6, **dadurch gekennzeichnet, dass** man
- das Kompartiment (3) in an sich bekannter Weise herstellt und dessen hautseitige Klebefläche mit einer allseitig um mindestens 4 mm überstehenden wiederablösbaren Schutzschicht (6) abdeckt,
- den Hauttyp des Patienten beispielsweise nach W. E. Roberts klassifiziert und mit Hilfe dieser Klassifikation dann aus nicht amin-resistenten, stark wasserdampfdurchlässigen Polysiloxanen, bei welchen nach der Polykondensation des Harz-Anteils und der Polydimethylsiloxanolgruppen die noch freien restlichen Silanolgruppen nicht mit Methylgruppen "gecapped" wurden diejenigen Polymere auswählt und gegebenenfalls aus den in den vorangehenden Ansprüchen beanspruchten Zusätzen diejenigen Zusätze auswählt, die erforderlich sind, damit das medizinische Produkt für eine Fixierdauer von mindesten 7 Tagen genutzt werden kann und dabei den Anforderungen an eine gute Hautverträglichkeit gerecht wird,
- daraus Beschichtungsmasse herstellt und diese auf einen Zwischenträger beschichtet und trocknet,
- die Rückschicht (1) auf das so erhaltene Laminat aus Schicht (2) und Zwischenträger auflaminiert und daraus ein Überpflaster einer solchen Größe und Form ausstanzt, welches das Kompartiment (3) allseitig um mindestens 4 mm überragt, und
- das so erhaltene Überpflaster schließlich nach Abziehen des Zwischenträgers auf die von der hautseitigen Klebefläche abgewandte Seite des Kompartiments (3) so aufklebt, dass sie dieses das Kompartiment allseitig um mindestens 4 mm überragt.

8. Kit-of-parts, umfassend
- ein auf der hautseitigen Klebefläche mit einer wiederablösbaren Schutzschicht (6) versehenes Kompartiment (3), das wie in einem der Ansprüche 1 bis 4 definiert ist, und
- mindestens ein auf einen Zwischenträger auflaminiertes wirkstofffreies Überpflaster aus
a) einer wasserdampfdurchlässigen Rückschicht (1) und
b) einer haftklebenden, nicht amin-resistente, stark wasserdampfdurchlässige Polysiloxanpolymere umfassende wirkstoffreie Polymerschicht (2), bei welchen nach der Polykondensation des Harz-Anteils und der Polydimethylsiloxanolgruppen die noch freien restlichen Silanolgruppen nicht mit Methylgruppen "gecapped" wurden, wobei das Überpflaster so groß ist, dass es das Kompartiment (3) allseitig überragt.

9. Kit-of-parts gemäß Anspruch 8, umfassend
- mindestens zwei auf einen Zwischenträger auflaminierte, wie im Anspruch 8 definierte wirkstofffreie Überpflaster, die sich hinsichtlich der Zusammensetzung der Schicht (2), ihrer Größe und/oder ihrer Form unterscheiden.

10. Kit-of-parts, umfassend
- mindestens zwei auf einen Zwischenträger auflaminierte wirkstofffreie Überpflaster aus
a) einer wasserdampfdurchlässigen Rückschicht (1) und
b) einer haftklebenden, nicht amin-resistente, stark wasserdampfdurchlässige Polysiloxanpolymere umfassende wirkstoffreie Polymerschicht (2), bei welchen nach der Polykondensation des Harz-Anteils und der Polydimethylsiloxanolgruppen die noch freien restlichen Silanolgruppen nicht mit Methylgruppen "gecapped" wurden wobei sich die Überpflaster hinsichtlich der Zusammensetzung der Schicht (2), ihrer Größe und/oder ihrer Form unterscheiden.

11. Kit-of-parts gemäß einem der Ansprüche 8 bis 10, in welchem die Schicht(en) (2) wie in Anspruch 5 definiert ist.

12. Verwendung eines Kit-of-parts gemäß einem der Ansprüche 8 bis 11 bei einem Verfahren zur Herstellung eines medizinisches Produkts, bestehend aus einem zentralen Kompartiment (3) und einem wirkstofffreien Überpflaster aus
a) einer wasserdampfdurchlässigen Rückschicht (1) und
b) einer haftklebenden, nicht amin-resistente, stark wasserdampfdurchlässige Polysiloxanpolymere umfassende wirkstoffreie Polymerschicht (2), bei welchen nach der Polykondensation des Harz-Anteils und der Polydimethylsiloxanolgruppen die noch freien restlichen Silanolgruppen nicht mit Methylgruppen "gecapped" wurden,
worin das Kompartiment (3) haftklebend ist und nach Abziehen der wiederablösbaren Schutzschicht (6) direkten Hautkontakt hat, bei welchem ein auf einen Zwischenträger auflaminiertes wirkstofffreies Überpflaster nach Abziehen des Zwischenträgers auf ein Kompartiment (3) aufgeklebt wird und dieses allseitig überragt.

13. Verwendung eines wie im Anspruch 1 definierten wirkstofffreien Überpflasters zur Verhinderung der Kristallisation des Wirkstoffs / der Wirkstoffe in der wirkstoffhaltigen Polymerschicht 5 eines TTS gemäß Anspruch 2, oder zur Verminderung des kalten Flusses in einem TTS gemäß Anspruch 2.

## Claims

1. Medical product consisting of a central compartment (3) and an active-ingredient-free overplaster composed of
a) a water vapor-permeable backing layer (1) and
b) a pressure-sensitively adhering, active-ingredient-free polymer layer (2),
wherein the compartment (3) is pressure-sensitively adhering and has direct skin contact after removal of the redetachable protective layer (6),
**characterized in that** the overplaster fixes the central compartment (3) and overhangs it on all sides and **in that** the active-ingredient-free polymer layer (2) comprises non-amine-resistant polysiloxane polymers in which, after the polycondensation of the resin fraction and of the polydimethylsiloxanol groups, the remaining silanol groups still free have not been capped by methyl groups.

2. Medical product according to Claim 1, **characterized in that** it is a transdermal therapeutic system (TTS) wherein the compartment (3) consists of
a) at least one pressure-sensitively adhering, active-ingredient-containing polymer layer (5) which has direct skin contact after removal of the redetachable protective layer (6), and
b) a separating layer (4), preferably of polyester, which covers the layer (5).

3. Medical product according to Claim 1, **characterized in that** it is a diagnostic agent and the diagnosis apparatus is located in the compartment (3).

4. Medical product according to Claim 1, **characterized in that** it is a cannula fixing plaster and the cannula is located in the compartment (3).

5. Medical product according to any of Claims 1 to 4, **characterized in that** the layer (2) comprises 0 to 4 % (w/w) of silicone oil, based on the dry weight of this layer.

6. Medical product according to any of Claims 1 to 5, **characterized in that** the backing layer (1) comprises an elastic fabric permeable to water vapor, preferably a polyester fabric.

7. Method for producing a medical product according to any of Claims 1 to 6, **characterized in that**
- the compartment (3) is produced conventionally and its skin-side adhesive face is lined with a redetachable protective layer (6) which protrudes on all sides by at least 4 mm,
- the patient's skin type is classified by the method, for example, of W. E. Roberts, and then, with the aid of this classification, a selection is made, from non-amine-resistant, highly water vapor-permeable polysiloxanes, in which, after the polycondensation of the resin fraction and of the polydimethylsiloxanol groups, the remaining silanol groups still free have not been capped by methyl groups, of those polymers - and, optionally, from the additions claimed in the preceding claims, a selection is made of those additions - which are necessary in order for the medical product to be able to be utilized for a fixing time of at least 7 days while meeting the requirements for good skin compatibility,
- coating material is produced therefrom and is coated onto an intermediate carrier and dried,
- the backing layer (1) is laminated onto the resulting laminate of layer (2) and intermediate carrier, and from this laminate an overplaster is punched out in a size and shape which overhangs the compartment (3) on all sides by at least 4 mm, and
- the resulting overplaster, finally, after removal of the intermediate carrier, is adhered to the side of the compartment (3) that is facing away from the skin-side adhesive face, in such a way that it overhangs this compartment on all sides by at least 4 mm.

8. Kit-of-parts comprising
- a compartment (3) which is provided on the skin-side adhesive face with a redetachable protective layer (6), the compartment (3) being as defined in any of Claims 1 to 4, and
- at least one active-ingredient-free overplaster which is laminated to an intermediate carrier and is composed of
a) a water vapor-permeable backing layer (1) and
b) a pressure-sensitively adhering, active-ingredient-free polymer layer (2) comprising non-amine-resistant, highly water vapor-permeable polysiloxane polymers, in which, after the polycondensation of the resin fraction and of the polydimethylsiloxanol groups, the remaining silanol groups still free have not been capped by methyl groups, the overplaster being large enough to overhang the compartment (3) on all sides.

9. Kit-of-parts according to Claim 8, comprising
- at least two active-ingredient-free overplasters defined as in Claim 8 and laminated onto an intermediate carrier, said overplasters differing in terms of the composition of the layer (2), their size and/or their shape.

10. Kit-of-parts comprising
- at least two active-ingredient-free overplasters laminated onto an intermediate carrier and composed of
a) a water vapor-permeable backing layer (1) and
b) a pressure-sensitively adhering, active-ingredient-free polymer layer (2) comprising non-amine-resistant, highly water vapor-permeable polysiloxane polymers, in which, after the polycondensation of the resin fraction and of the polydimethylsiloxanol groups, the remaining silanol groups still free have not been capped by methyl groups, where the overplasters differ in terms of the composition of the layer (2), their size and/or their shape.

11. Kit-of-parts according to any of Claims 8 to 10, in which the layer (s) (2) is (are) defined as in Claim 5.

12. Use of a kit-of-parts according to any of Claims 8 to 11 in a method for producing a medical product consisting of a central compartment (3) and an active-ingredient-free overplaster composed of
a) a water vapor-permeable backing layer (1) and
b) a pressure-sensitively adhering, active-ingredient-free polymer layer (2) which is not amine-resistant and which comprises highly water vapor-permeable polysiloxane polymers, in which, after the polycondensation of the resin fraction and of the polydimethylsiloxanol groups, the remaining silanol groups still free have not been capped by methyl groups,
wherein the compartment (3) is pressure-sensitively adhering and has direct skin contact after removal of the redetachable protective layer (6), in which an active-ingredient-free overplaster laminated onto an intermediate carrier is adhered, following removal of the intermediate carrier, to a compartment (3) and overhangs it on all sides.

13. Use of an active-ingredient-free overplaster as defined in Claim 1 for preventing the crystallization of the active ingredient/ingredients in the active-ingredient-containing polymer layer 5 of a TTS according to Claim 2, or for reducing the cold flow in the TTS according to Claim 2.

## Revendications

1. Produit médical, constitué d'un compartiment central (3) et d'un pansement de recouvrement sans substance active, constitué de
a) une couche arrière (1) perméable à la vapeur d'eau et
b) une couche polymère (2) adhésive, sans substance active,
dans lequel le compartiment 3 est adhésif et, après retrait de la couche protectrice (6) détachable, a un contact direct avec la peau,
**caractérisé en ce que** le pansement de recouvrement fixe le compartiment central (3) et déborde de celui-ci de tous les côtés et **en ce que** la couche polymère (2) sans substance active comprend des polymères de polysiloxane non amino-résistants, tels que, après la polycondensation de la part de résine et des groupes polydiméthylsiloxanol, les groupes silanol restants encore libres n'ont pas été "cappés" avec des groupes méthyle,

2. Produit médical selon la revendication 1, **caractérisé en ce qu'**il s'agit d'un système thérapeutique transdermique (TTS), dans lequel le compartiment (3) est constitué de
a) au moins une couche polymère (5) qui est adhésive et contient une substance active et qui, après retrait de la couche protectrice (6) détachable, a un contact direct avec la peau, et
b) une couche séparatrice (4), recouvrant la couche (5), de préférence en polyester.

3. Produit médical selon la revendication 1, **caractérisé en ce qu'**il s'agit d'un système de diagnostic et que le dispositif de diagnostic est localisé dans le compartiment (3).

4. Produit médical selon la revendication 1, **caractérisé en ce qu'**il s'agit d'un pansement de fixation de canule et que la canule est localisée dans le compartiment (3).

5. Produit médical selon l'une quelconque des revendications 1 à 4, **caractérisé en ce que** la couche (2) contient 0 à 4 % (w/w) d'huile de silicone, par rapport au poids sec de cette couche.

6. Produit médical selon l'une quelconque des revendications 1 à 5, **caractérisé en ce que** la couche arrière (1) comprend un tissu élastique, perméable à la vapeur d'eau, de préférence un tissu en polyester.

7. Procédé de fabrication d'un produit médical selon l'une quelconque des revendications 1 à 6, **caractérisé en ce que**
- on fabrique le compartiment (3) de manière connue et on couvre sa surface adhésive côté peau avec une couche protectrice (6) détachable débordant d'au moins 4 mm de tous les côtés,
- on classe le type de peau du patient par exemple selon W. E. Roberts et, à l'aide de cette classification, on choisit ensuite parmi des polysiloxanes non amino-résistants et très perméables à la vapeur d'eau, dans lesquels, après la polycondensation de la part de résine et des groupes polydiméthylsiloxanol, les groupes silanol restants encore libres n'ont pas été "cappés" avec des groupes méthyle, ceux des polymères et le cas échéant parmi les produits ajoutés revendiqués dans les revendications précédentes ceux des produits ajoutés qui sont nécessaires pour que le produit médical puisse être utilisé pour une durée de fixation d'au moins 7 jours et qu'il réponde ce faisant aux exigences d'une bonne tolérance cutanée,
- on fabrique à partir de là une pâte de revêtement et on l'applique et la sèche sur un support intermédiaire,
- on lamine la couche arrière (1) sur le produit laminé ainsi obtenu à partir de la couche (2) et du support intermédiaire et on découpe à partir de là un pansement de recouvrement d'une dimension et d'une forme telles qu'il déborde d'au moins 4 mm du compartiment (3) de tous les côtés, et
- on colle finalement le pansement de recouvrement ainsi obtenu, après retrait du support intermédiaire, sur celui des côtés du compartiment (3) qui est à l'opposé de la surface adhésive côté peau de telle sorte qu'il déborde d'au moins 4 mm du compartiment de tous les côtés.

8. Kit d'éléments, comprenant
- un compartiment (3) qui est muni d'une couche de protection (6) détachable sur la surface adhésive côté peau et qui est défini comme dans l'une quelconque des revendications 1 à 4, et
- au moins un pansement de recouvrement sans substance active, laminé sur un support intermédiaire, constitué de
a) une couche arrière (1) perméable à la vapeur d'eau et
b) une couche polymère (2) adhésive, sans substance active, comprenant des polymères de polysiloxane non amino-résistants et très perméables à la vapeur d'eau, tels que, après la polycondensation de la part de résine et des groupes polydiméthylsiloxanol, les groupes silanol restants encore libres n'ont pas été "cappés" avec des groupes méthyle, le pansement de recouvrement étant d'une dimension telle qu'il déborde du compartiment (3) de tous les côtés.

9. Kit d'éléments selon la revendication 8, comprenant
- au moins deux pansements de recouvrement sans substance active qui sont laminés sur un support intermédiaire et définis comme dans la revendication 8 et qui se distinguent par la composition de la couche (2), leur dimension et/ou leur forme.

10. Kit d'éléments, comprenant
- au moins deux pansements de recouvrement sans substance active et laminés sur un support intermédiaire, constitués de
a) une couche arrière (1) perméable à la vapeur d'eau et
b) une couche polymère (2) adhésive, sans substance active, comprenant des polymères de polysiloxane non amino-résistants et très perméables à la vapeur d'eau, tels que, après la polycondensation de la part de résine et des groupes polydiméthylsiloxanol, les groupes silanol restants encore libres n'ont pas été "cappés" avec des groupes méthyle, dans lequel les pansements de recouvrement se distinguent par la composition de la couche (2), leur dimension et/ou leur forme.

11. Kit d'éléments selon l'une quelconque des revendications 8 à 10, dans lequel la ou les couches (2) sont définies comme dans la revendication 5.

12. Utilisation d'un kit d'éléments selon l'une quelconque des revendications 8 à 11 dans un procédé de fabrication d'un produit médical constitué d'un compartiment central (3) et d'un pansement de recouvrement sans substance active, constitué de
a) une couche arrière (1) perméable à la vapeur d'eau et
b) une couche polymère (2) adhésive, sans substance active, comprenant des polymères de polysiloxane non amino-résistants, très perméables à la vapeur d'eau, tels que, après la polycondensation de la part de résine et des groupes polydiméthylsiloxanol, les groupes silanol restants encore libres n'ont pas été "cappés" avec des groupes méthyle,
dans lequel le compartiment (3) est adhésif et, après retrait de la couche protectrice (6) détachable, a un contact direct avec la peau et dans lequel un pansement de recouvrement sans substance active, laminé sur un support intermédiaire, est collé, après retrait du support intermédiaire, sur un compartiment (3) et déborde de celui-ci de tous les côtés.

13. Utilisation d'un pansement de recouvrement sans substance active, défini comme dans la revendication 1, pour empêcher la cristallisation de la ou des substances actives dans la couche polymère (5), contenant des substances actives, d'une TTS selon la revendication 2, ou pour réduire le flux froid dans un TTS selon la revendication 2.
